# EUROPEAN PATENT APPLICATION

(11) **EP 2 329 817 A1**
(43) Date of publication of application: **08.06.2011**
(21) Application number: 09169518.9
(22) Date of filing: 04.09.2009
(51) Int. Cl.: A61K 31/382, C12N 15/113, A61P 35/00, A61P 35/04

(54) **Retinoic acid receptor antagonists, miR-10a inhibitors and inhibitors of HOXB1 or HOXB3 repressors for treating pancreatic cancer**

(71) Applicant: Ernst-Moritz-Arndt-Universität Greifswald, 17487 Greifswald (DE); Universiteit Leiden, 2311 EZ Leiden (NL)
(72) Inventor: Weiß, Frank Ulrich, 17489, Greifswald (DE); Bagowski, Christoph P., 2311 NT, Leiden (NL); Lerch, Markus M., 17489, Greifswald (DE)
(74) Representative: Wablat, Wolfgang

(57) **Abstract**

The present invention relates to RAR antagonists or miR-10a inhibitors or inhibitors of HOXB1 and/or HOXB3 repressors for the treatment of pancreatic cancer.

## Description

Pancreatic cancer is a highly invasive malignancy which is characterised by early metastasis formation. Although metastases spawned by malignant tumours are responsible for over 90% of all cancer deaths¹, our understanding of the mechanisms involved in metastasis formation is still rather limited.

There is increasing evidence that microRNAs can function as oncogenes or as tumour suppressors and that differential expression of specific microRNAs is critically involved in the progression of many cancers²⁻⁵. Large scale transcriptome analysis data are available for microRNA signatures of different human tumours⁶⁻⁷, including pancreatic cancer^{8,9}. These studies used microarray technology to reveal specific microRNA expression profiles in specimen from pancreatic adenocarcinoma, which could be differentiated from normal pancreatic tissue and chronic pancreatitis.

In a recent publication, it was shown that miR-10b is highly expressed in human breast tumours and that expression correlates with metastatic potential^{10..} In pancreatic cancer miR-10b expression is not elevated, but instead an upregulated expression of the related miR-10a has been described^{8,9}. Both microRNAs belong to the same microRNA family, but miR-10b is located in the HOXD cluster on chromosome 2, whereas miR-10a is present in the HOXB cluster on chromosome 17¹¹. Since related microRNAs are expected to share similar target genes, we hypothesized that elevated miR-10a levels in pancreatic tumours could be involved in their metastatic behaviour.

It is known that many of the 39 mammalian Homeobox genes are regulated by retinoids through retinoic acid response elements (RARE) and miR-10a is located in the 3'genomic region of the well known RA target HOXB4, in the vicinity of a RARE¹². Retinoids (retinoic acid (RA) and its derivatives) regulate a wide variety of different biological processes during development. Experimentally applied retinoid agonists and antagonists promote differentiation, inhibit proliferation and accordingly show promise for the prevention and treatment of cancer¹³.

The problem underlying the present invention was the provision of new agents for the treatment of pancreatic cancer. This problem was solved by RAR antagonists or miR-10a inhibitors or inhibitors of HOXB1 and/or HOXB3 repressors for the treatment of pancreatic cancer.

Especially, the problem was solved by RAR antagonists or miR-10a inhibitors or inhibitors of HOXB1 and/or HOXB3 repressors for the prevention or reduction of metastasis in pacreatic cancer. "Reduction" means a reduction of invasiveness and metastatic behaviour of between 20 and 99%, preferably between 30 and 95%.

Herein, we investigated the importance of miR-10a in pancreatic cancer metastasis and the possible link with retinoic acid. We show that miR-10a expression promotes metastatic behaviour of pancreatic tumour cells and that repression of miR-10a is sufficient to inhibit invasion and metastasis formation. We further demonstrate that miR-10a is a retinoid acid target and that retinoic acid receptor (RAR) antagonists effectively repress miR-10a expression and inhibit metastasis. We find that miR-10a downstream targets, HOXB1 and HOXB3 are involved in promoting metastatic behaviour and that the anti-metastatic activity of RAR antagonists can be prevented by specific knock down of HOXB1 and/or HOXB3. Our findings show that both, miR-10a inhibitors and RAR antagonists are effective compounds for targeting metastatic tumour behaviour through regulating miR-10a function and HOXB1 and/or HOXB3 expression. Interestingly, suppression of HOXB1 and HOXB3 in pancreatic cancer cells is sufficient to promote metastasis formation.

These findings indicate that miR-10a is a key mediator of metastatic behaviour in pancreatic cancer which regulates metastasis via suppression of HOXB1 and HOXB3. Inhibition of miR-10a expression (with RAR antagonists) or function (with specific inhibitors) is a starting point for anti-metastatic therapies.

Preferred RAR anatgonists are Ro-41-5253 (also known as LG-629, CID5312120, Benzoic acid, 4-(2-(7-(heptyloxy)-3,4-dihydro-4,4-dimethyl-2H-1-benzothiopyran-6-yl)-1-propenyl)-, S,S-dioxide, (E)) and its derivatives. "Derivatives" means related (natural and synthetic) benzothiopyran compounds with preferential antangonistic function on retinoic acid receptor-alpha.
Further preferred RAR antagonists are selected from the group of retinoid-like tricyclic compounds (e.g. dihydranthracenyl, benzchromenyl, benzothiochromenyl retinoids) which bind with high affinity to RARs and are potent antagonists of retinoic acid function in vitro and in vivo systems.

A preferred miR-10a inhibitor is the miR-10a antisense sequence CACAAATTCGGATCTACAGGGTA (Morpholino; Morpholino antisense oligonucleotide, miR-10a antisense Morpholino sequence). Other nucleic acid sequences, wherein one or more of these 23 nucleic acids are deleted or substituted or wherein one or more nucleic acids are added and which still inhibit the miR-10a sequence are also part of the invention.

Suitable inhibitors of HOXB1 and HOXB3 repressors are selected from the group of antisense tools such as siRNA, Morphilinos, in vivo Morpholins which are able to prevent down regulation of HOXB1 and/or HOXB3 expression.

We identified and validated miR-10a as a novel gene with an important function in tissue invasion and metastasis formation. Overexpression of miR-10a siRNAs alone suffices to acquire invasiveness and metastatic behaviour in otherwise non-metastatic pancreatic cancer cells, whereas silencing miR-10a with gene specific Morpholinos blocks metastatic behaviour of metastatic pancreatic cancer cells and primary human tumours. We demonstrate that specific miR-10a inhibitors can be used to prevent metastatic behaviour of xenotransplanted resection specimen of human pancreatic tumours in zebrafish embryos. Morpholinos can remain functional for several days³⁴ and recently "*in vivo* Morpholinos", which are covalently linked to a delivery component, have become available (Gene Tools, LLC).
The early embryos used here (2dpf) do not reject these primary tumour xenografts, due to the fact that their immune system is not fully developed. Major maturation events leading to immunocompetence occur in the zebrafish between 2 and 4 weeks post fertilisation (wpf), coinciding with the larval to juvenile transitory phase ³⁵.

The identification of upstream regulators of miR-10a in pancreatic cancer cells is of importance. We show that miR-10a is regulated by RA signalling in pancreatic cancer cells. Retinoids have been used as chemopreventive and anticancer agents because of their pleiotropic regulator function in cell differentiation, growth, proliferation and apoptosis, however to date limited amount of information on the role of retinoids or RAR antagonists in metastasis formation is available. For pancreatic cancer we clearly demonstrate anti-metastatic properties of RARAα antagonists, which downregulate miR-10a, and therefore may have potential as anti-metastatic drugs. Whether this role of miR-10a and RARs is specific for pancreatic cancer or a common mechanism in other cancer types, as for example shown for an Ampulla vateri tumour, will need to be determined in more detail.
Notably we found upregulated miR-10a expression already in conditions of chronic pancreatitis. It is well recognized that chronic conditions of pancreatitis represent a risk factor for the development of pancreatic cancer³⁶. However, the route from inflammation to cancer involves many steps and general observations concerning the risk of pancreatic adenocarcinoma in patients with CP suggest an accelerated process of mutagenesis and mutation accumulation rather than a unique process. The specific role of miR-10a in the progression from chronic pancreatitis to cancer cannot be clarified at this time. Upregulation of miR-10a expression could be involved in a more complex regulation mechanism that controls or parallels the malignant transformation of a cell. So miR-10a might regulate additional early steps in tumor progression which are not related to cell migration, so that additional triggers are necessary for the initiation of the metastatic phenotype which are absent in chronic pancreatitis, but are active in transformed carcinoma cells.

We identified here downstream targets of miR-10a, namely HOXB1 and HOXB3 and the cadherin/catenin complex proteins E-cadherin, α-catenin and β-catenin. Results from a target scan analysis (TargetScan Software, release 5.1 see homepage http.www.targetscan.org/) identified an 8mer seed match of miR-10a within the 3'UTR region of HOXB3at position 859-865. Our results indicate that HOXB1 and HOXB3 function as metastasis suppressor genes. Whether downregulation of cadherin/catenin proteins is controled via suppression of HOXB1 and HOXB3 or occurs through a parallel signalling pathway will have to be further analysed.

In summary, we identified miR-10a as a RA responsive mediator of invasiveness and metastatic behaviour in pancreatic cancer which promotes its effects through HOXB1 and HOXB3 suppression. Inhibition of miR-10a expression or function represents a promising strategy for anti-metastatic therapy and both RAR antagonists, as well as miR-10a inhibitors could be powerful anti-metastatic compounds.

### Examples

### Materials and methods

### Animal care and handling

Zebrafish (Danio rerio) (Tuebingen line, AIB strain (Albinos) and Tg (fli1:eGFP) were handled in compliance with local animal care regulations and standard protocols of the Netherlands and Germany. Fish were kept at 28°C in aquaria with day/night light cycles (10 h dark versus 14 h light periods). The developing embryos were kept in an incubator at constant temperatures.

### Retinoic acid and RA Inhibitors

The selective RARα antagonist Ro-41-5253 was purchased from Biomol International, dissolved in DMSO and cells were treated overnight prior to injections at a final concentration of 200 nM (50% inhibition at 60nM)^{14,15}. Retinoic acid (ATRA, Sigma) was added to cells ON at a final concentration of 1 µM.

### Cell Culture

Cell lines PaTu8988T, PaTu8988S, AsPC1, Capan1, Capan2, MiaPaCa2, PANC1 and PaTu8902 cells were cultured in DMEM high glucose, with 10% FCS and 1:100 Pen/Strep.

### Cell Staining, Injections, Incubations and Antibodies

Cells were stained with CM-Dil (red fluorescence) at a 1:1000 dilution according to the manufacturers protocol (Vybrant, Invitrogen). Cells were suspended in 67% DPBS for injection into embryos. 2dpf zebrafish embryos were dechorionated and tranquilized with tricaine (Sigma). An injection needle (15µm diameter) with a manual injector (Eppendorf; Celltram Vario) was used to inject ∼200 cells into the yolk sac of 2dpf zebrafish embryos. On average 80 embryos were injected for each treatment and embryos with detectable cell scatter 2 h after the transfer (and therefore presumably due to the transfer procedure) were excluded from further analysis. Presence of cells was checked after 2 h incubation at 31°C and embryos were subsequently kept at 35°C. We defined metastasis based on the number of fluorescent cells that were detected outside the yolk sac 2 days after the transfer. Presence of 5 or more cells was considered "metastasised" and percentages of metastasised fish in each experiment were counted. The occurence of micro-metastasis is indicated by cell clusters of ≥4 cells presumably originating from cell division.
PaTu-S cells and ASPC1 cells were transfected ON with indicated concentrations of miR-10a siRNA in the presence of ICA-Fectin TM442 (Eurogentec, Belgium). Whole cell lysates were prepared 36h post-transfection and 40 µg of total lysates were separated on 7.5% PAA-gels and blotted with anti E-cadherin, α-catenin, β-catenin (all mAbs, Transduction Labs) and actin antibodies (mAb Sigma-Aldrich, Germany).

### Tissue preparation for transplantation into zebrafish embryos

Human resection specimens were obtained at the Universitätsklinikum Greifswald according to local ethical guidelines and after obtaining informed patient consent. Tumour tissue was cut into small pieces using a scalpel blade. Tissue fragments were then washed with 67% DPBS and stained with 1:500 CM-Dil. Stained tissue fragments of approximal ¹/₅ to ½ the size of the yolk sac were transferred with a glass transplantation needle into the yolk. After 2 h at 31 °C the embryos were checked that the yolk had sealed itself and the embryos were incubated at 35°C for the remainder of the experiment.

### Nothern Blot, RT-PCR and quantitative RT-PCR (RT-qPCR)

Northern Blot analysis was performed essentially according to Kloosterman et al. ¹⁶ using a miR-10 LNA probe (CACAAATTCGGATCTACAGGGTA) (Exiqon, Denmark). We had previously described protocols for RT-PCR¹⁷ and RT-qPCR¹⁸ . Primers for the RT-PCR were: HOXB1: sc-38686 and HOXB3: sc-38690-PR and RARα: sc-29465-PR all from Santa Cruz Biotechnology. For quantitative RT-PCR we used 100 ng of total RNA and the Roche Master SYBR Green kit. Single-band amplification was verified by melting curve dissociation protocols. A standard curve for β-actin using 1, 5, 10, 100, and 500 ng of total RNA was used for normalization. RT-qPCR primers for HOXB1 were: forward 5'-ctccgaggacaaggaaacac-3' and reverse 5'-ccttcatccagtcgaaggtc-3' and for HOXB3: forward 5'-actccaccctcaccaaacag-3' and reverse 5'-ttgcctcgactctttcatcc-3'.

### Morpholino knockdown, siRNA expression and Sensor Construct

Morpholino antisense oligonucleotides were obtained from Gene Tools, LLC (OR, USA) and used as previously described¹⁹. The miR-10a antisense Morpholino sequence is: CACAAATTCGGATCTACAGGGTA. Standard control Morpholino was CCTCTTACCTCAGTTACAATTTATA. Both miR-10a and control Morpholinos were fluorescein labeled. The siRNAs for human HOXB1, human HOXB3 and human RARα were purchased from Santa Cruz Biotechnology (HOXB1: sc-38686, HOXB3: sc-38690 and RARα: sc-29465). EndoPorter (Gene Tools, LLC) was used to deliver Morpholinos and siRNAs into cells and efficiencies for Morpholinos were checked by fluorescence microscopy to be >98%. For the miR-10a siRNA, the sense strand corresponds to UACCCUGUAGAUCCGAAUUUGUGUG. The sequence of control siRNA (miR-196a) was UAGGUAGUUUCAUGUUGUUGG with the corresponding antisense sequence). 30 µl of each siRNA oligo (50 µM) were annealed by gradually cooling from 98°C to 20°C in a volume of 75 µl annealing buffer (50 mM Tris, pH 7.8, 100 mM NaCl RNAse free). The effect of miR-10a Morpholinos on miR-10a function was tested with a sensor construct: pCS2+ EGFP 4x miR-10 AS containing 4 copies of a miR-10a antisense sequence.

**Statistical Analysis** Significance between groups was assessed by Student's t-test.

**Imaging** Confocal pictures were taken with the Biorad Confocal microscope 1024ES (Zeiss microscope) combined with Krypton/Argon laser, a dual laser scanning confocal microscope Leica DM IRBE (Leica) or with the Nikon TE300 confocal microscope and a coherent Innova 70C laser (Chromaphor, Duisburg, Germany). Pictures were further taken in a differential interference contrast (DIC) microscope (Axioplan 2) with an AxioCam MR5 camera (Carl Zeiss). For fluorescent stereomicroscope images we used a Leica MZ16FA microscope and a Leica DFC 420C camera.

### Results

### miR-10a expression is elevated in pancreatic tumour cell lines and specimen of chronic pancreatitis and pancreatic tumours

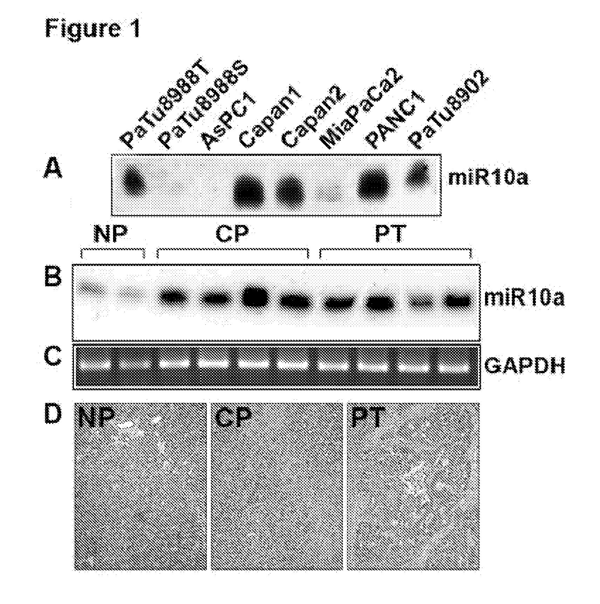

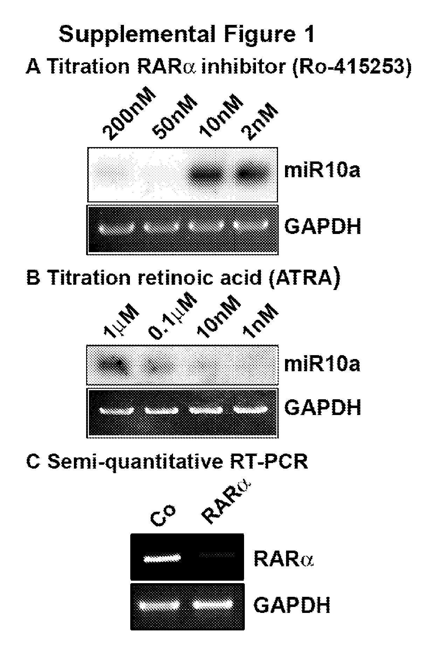

We performed northern blot analysis in a series of pancreatic tumour cell lines including PaTu8988-S and PaTu8988-T ²⁰(PaTu-S and PaTu-T), and observed miR10a expression in all but two (PaTu-S and AsPC1) cell lines (Fig.1 A). Expression differences in PaTu-S and PaTu-T cells are interesting as both sister cell lines originate from liver metastases of the same human pancreatic adenocarcinoma²⁰ and we and others have previously shown that E-Cadherin expression in PaTu-S cells correlates with the maintenance of functional cell-cell contacts and a reduced tendency of cells to ²¹⁻²³ migrate. In PaTu-T cells, loss of E-Cadherin is associated with little cell aggregation and enhanced migratory capabilities, which we confirmed in a zebrafish xenotransplantation model ²³. These different migratory capacities seem to correlate with an elevated miR-10a expression in PaTu-T cells and a very low expression in PaTu-S cells (Fig.1 A).

We also assessed the expression of miR-10a in resection specimen of 4 pancreatic adenocarcinoma, 4 chronic pancreatitis and 2 normal pancreatic tissues and found a ∼2.8 fold upregulation of miR-10a expression in all 4 pancreatic tumours in comparison to control pancreatic tissue. In contrast to a previous microarray study⁹ we also observed increased miR-10a expression in chronic pancreatitis. (Fig.1 B).

### miR-10a is required and sufficient for tissue invasion and metastasis formation in pancreatic tumour cells

The zebrafish and its transparent embryos have recently been established as a model system to investigate tumour development ²⁴⁻²⁷. Xenotransplantation in fish is utilized to study invasiveness and metastatic behaviour of established cancer cells ^{23,25,27-31} and of fragments of resected gastrointestinal tumour tissue ²³.

To study the role of miR-10a in metastatic behaviour, we labelled pancreatic tumour cell lines with a carbocyanine dye (CM-Dil) and ectopically transplanted them into the yolk sac of 2 dpf zebrafish embryos. PaTu-S cells remained in the yolk, whereas PaTu-T cells invaded the embryo and showed metastatic behaviour (Table 1 and Supplemental Table 1). The expression of miR-10a in these cell lines therefore correlates with their metastatic potential in the zebrafish xenotransplantation model. We further tested 6 additional pancreatic tumour cell lines and found in most of them a correlation of metastatic behaviour with the presence of miR-10a but not with its absolute levels of expression (Fig.1A and Supplemental Table 1).

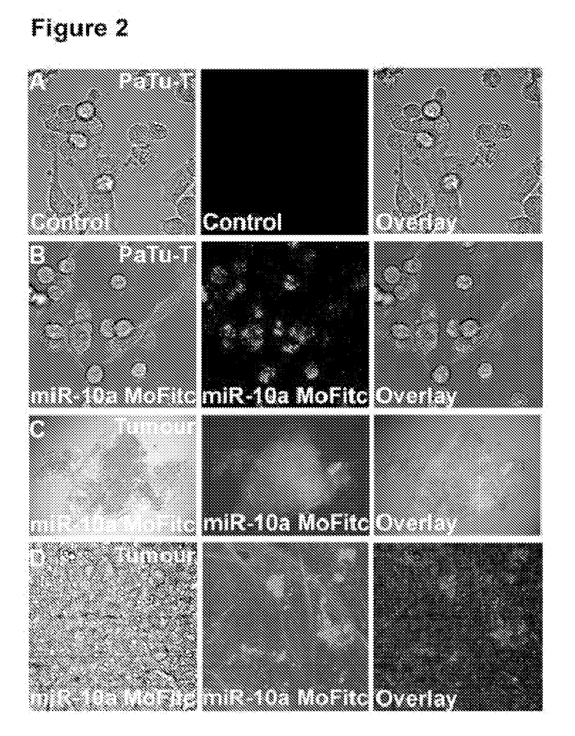

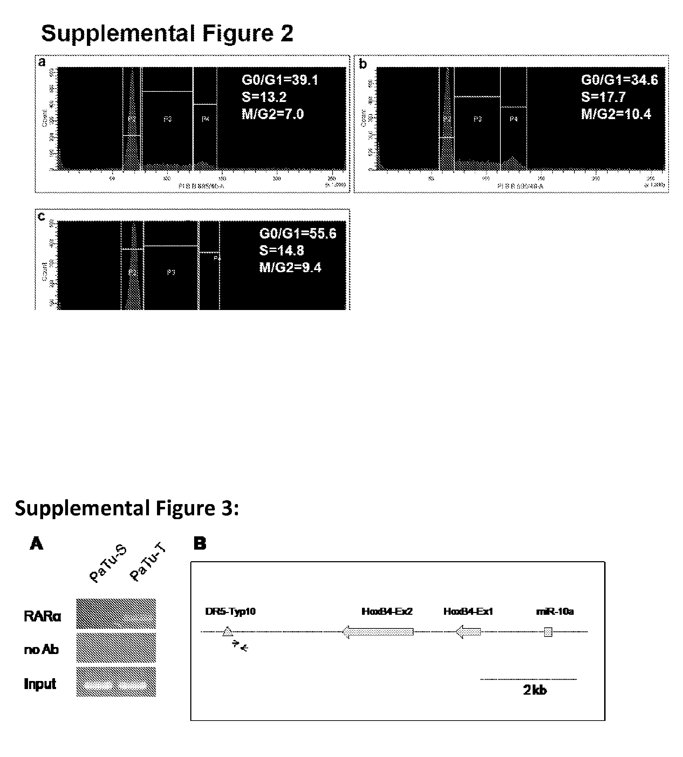

To investigate the involvement of miR-10a in the metastatic behaviour *in vivo,* we performed a loss of function analysis by silencing miR-10a with specific Morpholino antisense oligonucleotides which we previously have demonstrated to work well in silencing miR-10a in the developing zebrafish embryo ¹⁹. More than 98% of cancer cells incorporated the miR-10a Morpholino (Fig.2 B, FACS analysis; not shown) and also resection specimen of human tumour tissues showed significant uptake of Morpholino (Fig.2 C and D).

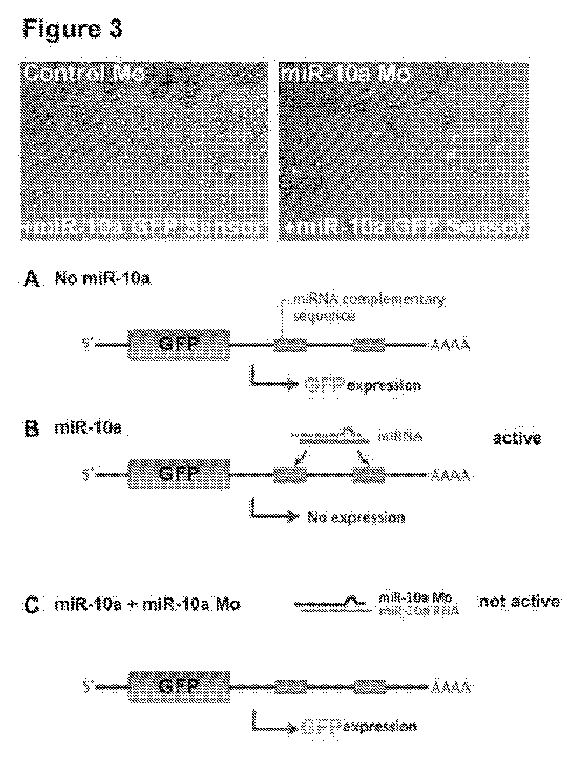

Expression of an EGFP miR-10a sensor construct, containing 4 miR-10a target sequences in the 3'UTR is repressed by the presence of the endogenous miR-10a in PaTu-T cells (the cell line highly expressing miR-10a) (Fig.3 left panel). Delivery of anti-miR-10a Morpholino to the cells hinders this repression, indicating that miR-10a Morpholinos work well in silencing miR-10a function (Fig.3 right panel).

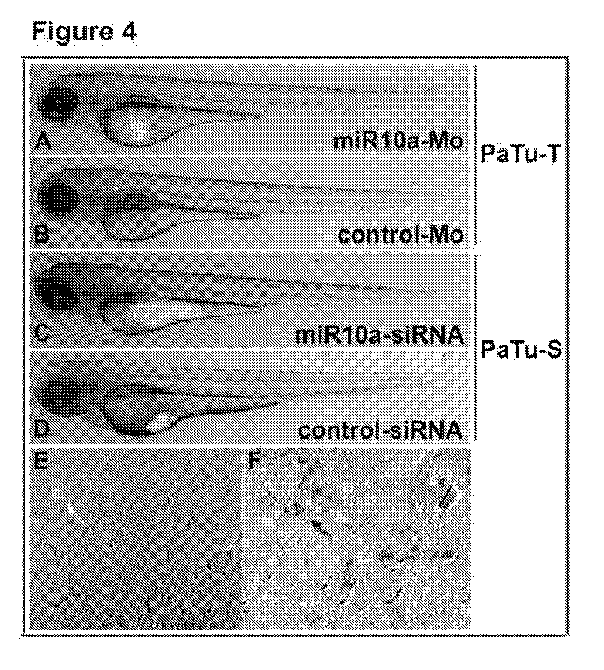

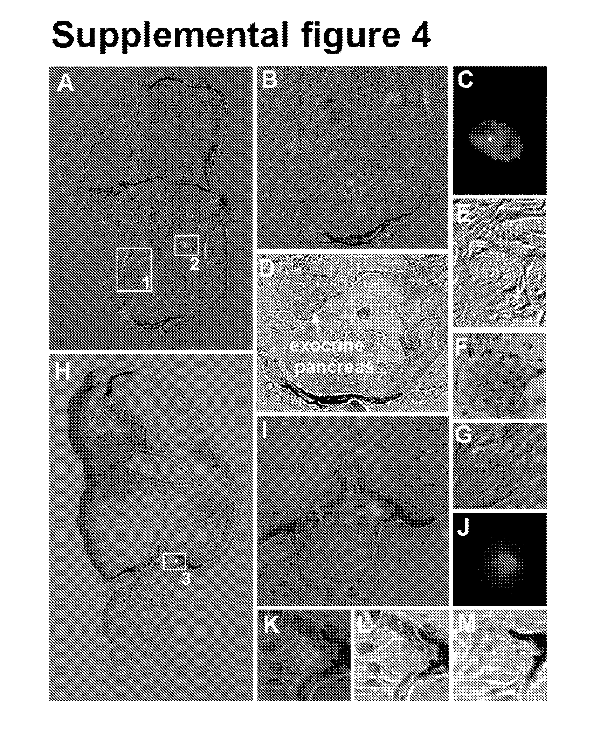

Targeting endogenous miR-10a with Morpholinos rendered PaTu-T-cells non-invasive and prevented metastatic behaviour in zebrafish embryos, whereas a control Morpholino had no such effect (Fig.4 B, Table1 and Supplemental Table 1). Over a three day time course we observed no adverse affects of Morpholino treatment on general cell survival. (analysed by propidium iodine (PI) staining, data not shown).

Complementary to this loss of 'metastatic' function, we also performed a gain of function analysis using miR-10a siRNA which can be effectively used to study miR-10a overexpression¹⁹. We transfected PaTu-S cells with miR-10a siRNAs and subsequently transplanted the cells into the yolk sac of 2 dpf embryos. The delivery of miR-10a siRNAs actually conferred invasiveness and metastasis formation to PaTu-S cells (39.2% (SD 7.4)) that were previously non-invasive *in vivo* (Fig.4 C). As a control we used miR-196a siRNAs, which showed no effect on cellular invasion and migration (0.57% (SD 1)) (Fig.4 D; Table 1 and Supplemental Table 1).

Our results indicate that expression of miR-10a in the cellular background of a pancreatic tumour cell is sufficient and also necessary to drive it's metastatic behaviour.

### miR-10a is required for invasion and metastatic behaviour of xenotransplanted primary human tumours

We investigated whether miR-10a gene-specific Morpholinos could also block metastatic behaviour of xenotransplanted primary human tumours. Small fragments of resected tumour tissue were treated with fluorescein labelled miR-10a Morpholinos (miR-10a Mo-FITC) and cellular uptake of miR-10a Mo-FITC was observed (Fig.2 C-D). Treatment of small tumour fragments from three different patients with miR-10a Mo prior to xenotransplantation led to over 90% reduction of invasion and metastatic behaviour, when compared to control Mo treated tissue of the same tumour specimen (Table 2 and Supplemental Table 1). Our results indicate a regulatory function of miR-10a in the metastatic behaviour of human pancreatic tumour cells (Note that one tumour was an ampulla vateri tumour, which "per definition" is not a pancreatic tumour. Still we also see inhibition of invasion in this tumour type).

### miR-10a expression is increased by retinoids and decreased by RAR antagonists in pancreatic tumour cells

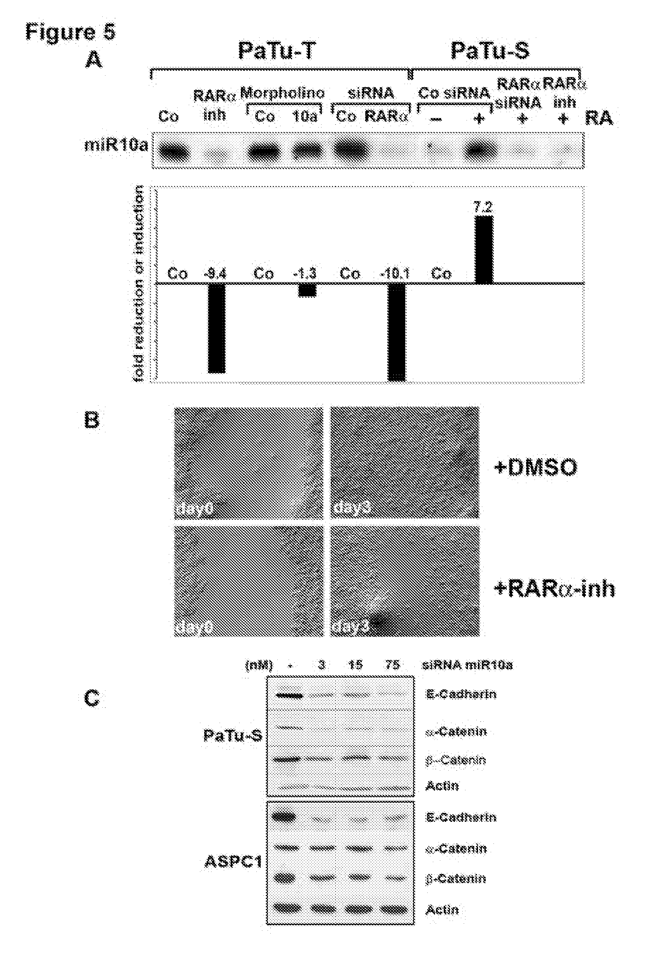

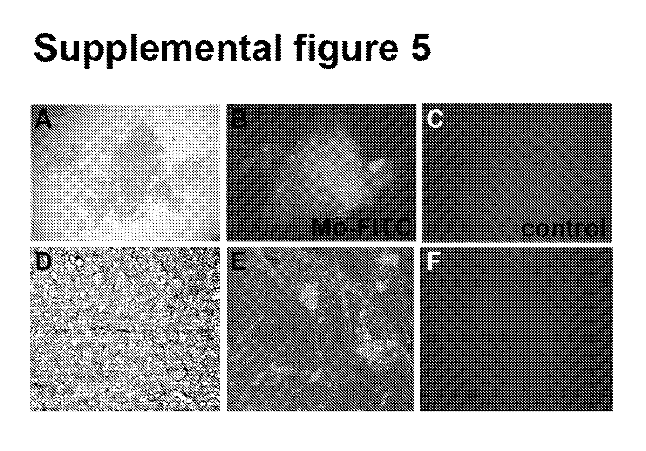

To analyse if miR-10a expression is under control of RA signalling in pancreatic cancer, we analyzed miR-10a expression in cells treated either with retinoids or a selective RARα antagonist. Our northern blot data show that miR-10a expression in non-metastatic PaTu-S cells is positively regulated by stimulation with all trans retinoic acid (ATRA) (Fig.5 A and Supplemental Fig.1), whereas a significant decrease in miR-10a expression was observed in the metastatic PaTu-T cells treated with either a selective RARα antagonist (Ro-41-5253; Biomol) or RARα-specific siRNAs (Fig.5 A and Supplemental Fig.1). Using chromatin immunoprecipitation (CHIP analysis), we identified the DR5 type 10 retinoic acid response element (RARE) as a functional target sequence for RARα binding in PaTu-T cells (but not in PaTu-S cells). This RARE has been previously identified as a mediator of hoxb4 neural expression³² and thus is likely to also regulate miR-10a (Supplemental Fig. 2).

### RARα inhibition prevents metastatic behaviour of pancreatic cancer cells and xenotransplanted tumours

We used the selective RARα antagonist Ro-41-5253; to analyze its anti-migratory activity in an *in vitro* migration assay ("scratch assay") and in the zebrafish xenotransplantation model, respectively. The RARα antagonist significantly decreased Patu-T cell migration in the *in vitro* assay (Fig.5 B) and inhibited invasion and metastatic behaviour in zebrafish embryos (Table 1 and Supplemental Table 1). Similar results were obtained when PaTu-T cells were treated with RAR -specific siRNAs (Supplemental Table 1 and Supplemental Fig. 1). We observed no adverse affects of RARα antagonist treatment on general cell survival (analysed by propidium iodine (PI) staining or cell cycle distribution (Supplemental Fig. 3).

Moreover, we treated small fragments of resected pancreatic tumours specimen with the selective RARα antagonist prior to xenotransplantation into zebrafish embryos. Compared to the DMSO treated control tissue of the same tumour specimen, we observed a strong reduction (> 95%) of invasiveness and metastatic behaviour (Table 2 and Supplemental Table 1).

### miR-10a regulates Cadherin/catenin expression

One of the known prerequisites for metastasis is the downregulation of cell-adhesion proteins e.g the proteins that constitute functional cadherin-catenin complexes ³³. We therefore analyzed if induction of migration in PaTu-S cells by miR-10a specific siRNAs affects expression of E-cadherin, α-catenin and β-catenin. We observed that miR-10a specific siRNAs clearly affected expression of all three proteins, however, to various extents (Fig. 5 C). Similar results were obtained in ASPC-1 cells that were shown to have no endogenous expression of miR-10a (Fig. 5 C). Our results indicate , that regulation of metastasis by miR-10a in pancreatic tumor cells involves down regulation of cadherin/catenin proteins which may enable the tumor cell to start migration.

### miR-10-a promotes invasiveness and metastatic behaviour through suppression of HOXB1 and HOXB3

In the zebrafish we have identified hoxB1 and hoxB3 as conserved downstream targets of the miR-10 family ¹⁹. Here we analysed if HOXB1 and HOXB3 are targets of miR-10a in human pancreatic cancer cells as well. We used siRNAs to specifically knock down HOXB1 and HOXB3 and found that suppression of either HOXB1 or HOXB3 conferred invasiveness and metastasis formation to otherwise non-metastatic PaTu-S cells (Table 1 and Supplemental Table 1). Simultanous suppression of HOXB1 and HOXB3 had an additive effect.

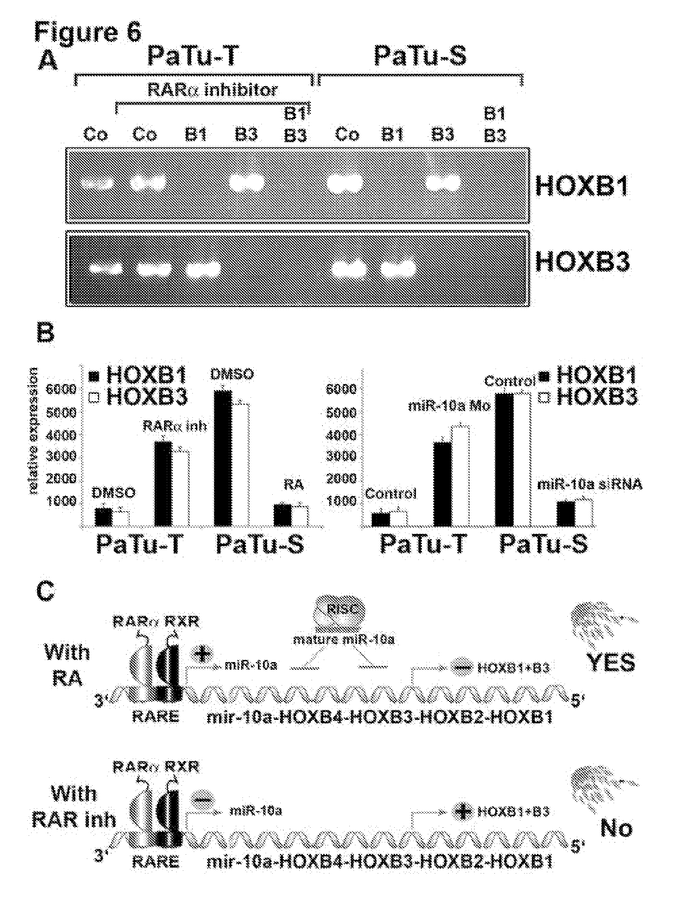

To examine whether HOXB1 and HOXB3 expression is required for the anti-metastatic effect of the RARα antagonist, we pretreated PaTu-T cells with both the RARα inhibitor and HOXB1 and HOXB3 specific siRNAs. Both siRNAs were functional and no transcripts were detectable by RT-PCR (Fig. 6 A). The anti-metastatic activity of RARα antagonist was repressed by HOXB1 or HOXB3 suppression in PaTu-T cells (Table 1 and Supplemental Table 1). Simultaneous suppression of HOXB1 and HOXB3 had an additive effect which annulled the anti-metastatic activity of the RARα antagonist. PaTu-T cells transfected with HOX siRNA or control siRNA were equally viable as tested by PI staining (data not shown). By quantitative PCR analysis we could show that RARα antagonist treatment increased HOXB1 and HOXB3 expression in PaTu-T cells and that in contrast RA treatment decreased the expression of both genes in PaTu-S cells (Fig. 6 B). These findings correlate with an inverse regulation of miR-10a expression, which is decreased by RARα antagonist and increased by RA stimulation (Fig.5 A). Furthermore, miR-10a overexpression in PaTu-S cells (by gene specific siRNAs) led to decreased expression of HOXB1 and HOXB3, whereas miR-10a knockdown led to increased expression of HOXB1 and HOXB3 in PaTu-T cells (Fig. 6 B). Taken together, these results strongly indicate that miR-10a exerts its role in metastasis formation through suppression of HOXB1 and HOXB3.

**Table 1**

| **paTU-T cells** | No treatment | Mo | +Control Mo | +RARα-inh | +RARα-inh HOXB1 siRN/\ | +RARα-inh HOXB3 siRNA | +RARα-inh HOXB1/B3 siRNlt |
|---|---|---|---|---|---|---|---|
| Average % | **38.2** | **37,7** | **2.9** | **2.2** | **25.3** | **25.4** | **45.8** |
| (SD) | (7) | (5.3) | (1.4) | (0.8) | (1.9) | (1.9) | (2.7) |
| | | | | | | | |

| **PaTa-S cells** | No treatment | +Control siRNA | +miR-10a siRNA | +RA | HOXB1 siRNA | HOXB3 siRNA | HOXB1/B3 siRNA |
|---|---|---|---|---|---|---|---|
| Average % | **0.3** | **0.5** | **39.2** | **27.7** | **31** | **29.9** | **52.5** |
| (SD) | (0.5) | (0.9) | (7.4) | (3,6) | (0,1) | (2.9) | (5,6) |

**Table 2**

| | Tumour Transplant (grade and pTMN stage) | Number transplants (survivors) Treatment | Migration [presence of micro-metastases at 3dpl] | | | total >5 [%] | Pancreatic Tumour Transplant | Number transplants (survivors) | Migration [presence of micro [presence of micro-metastases at 3dpl] | | | total >5[%] | p-value |
|---|---|---|---|---|---|---|---|---|---|---|---|---|---|
| | | | <5 | 5 to 20 | >20 | | | | <5 | 5 to 20 | >20 | | |
| 1 | Patient 1 G3; *T3*, *N1; M1* | 80 (70) Control Morpholino | 0 | 22 [8] | 14 [11] | 51.4% | Patient 1 | 80(64) miR-10a Morpholino | 1 [0] | 2 [0] | 0 | 3.1% | 23x10⁻⁵ |
| 2 | Patiant 2 G2; *T3; N1; M1* | 80 (62) Control Morpholino | 0 | 24 12 [6] | [10] | 58.1% | Patiant 1 | 80 (60) miR-10a Morpholino | 0 | 2 [0] | 0 | 3.3% | 6.3x10⁻⁵ |
| 3 | Patient 3 G2; *T3; N1; M1* | 80 (60) Control Morpholino | 0 | 22 [5] | 10 [8] | 49.3% | Patient 2 | 80 (66) miR-10a Morphotino | 0 | 4 [0] | 0 | 6.1% | 2.1x10⁻⁵ |
| 1 | Patient 1 G3; *T3. N1 M1* | 80 (71) DMSO | 0 | 21 [6] | 15 [11] | 50.7% | Patient 1 | 80 (65) RARα-inhibitor | 0 | 2 [0] | 0 | 3.1% | 4.8x10⁻⁵ |
| 2 | Pantient 2 G2; *T3; N1; M1* | 80 (72) DMSO | 0 | 27 [7] | 14 [11] | 50.9% | Patient 1 | 80 (70) RAPα-inhibitor | 1 | 3 [0] | 0 | 4.3% 4.4x10⁻⁵ | 1.6x10⁻⁵ |
| 3 | Patient 3 G2;*T3,N1, M1* | 80 (68) DMSO | 0 | 22 [6] | 12 [9] | 50% | Patient 2 | 80 (67) RARα-inhibitor | 0 | 3 [0] | 0 | 4.5% | 4.4x10⁻⁵ |

### Supplemental Table 1: Tables for all experiments

(including Tumor Transplants and PaTu-S and PaTu-T cells treated with siRNAs, Morpholinos, RA and RARα antagonist and additional human pancreatic cancer cell lines.)

### Morpholino treated Tumor Transplants

### Patient 1 Pancreatic Tumor

### poorly differentiated adenocarcinoma of the ampulla vateri with infiltration of the pancreas

### G3; pT3, pN1 mi (1/11) pM1

| **Control Morpholino** | | | | | | | **miR-10a Morpholino** | | | | | | |
|---|---|---|---|---|---|---|---|---|---|---|---|---|---|
| Day | Transplanted | Alive | Migration | | | **total >5** | Day | Transplanted | Alive | Migration | | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **70** | 0 | 22 | 14 | 36 | 1 | | **64** | 1 | 2 | 0 | 2 |
| 2 | | 59 | 0 | 19 | 14 | 33 | 2 | | 57 | 0 | 2 | 0 | 1 |
| 3 | | 53 | 0 | 18 | 12 | 30 | 3 | | 50 | 0 | 1 | 0 | 1 |
| | | | | | | **36 (51.4%)** | | | | | | | **2 (3.1 %)** |

### Patient 2 Pancreatic Tumor

### Well-differentiated adenocarcinoma of the pancreas

### G2; T3; N1; M1

| **Control Morpholino** | | | | | | | **miR-10a Morpholino** | | | | | | |
|---|---|---|---|---|---|---|---|---|---|---|---|---|---|
| Day | Transplanted | Alive | | Migration | | **total >5** | Day | Transplanted | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **62** | 0 | 24 | 12 | 36 | 1 | | **60** | 0 | 2 | 0 | 2 |
| 2 | | 59 | 0 | 20 | 15 | 35 | 2 | | 57 | 0 | 1 | 0 | 1 |
| 3 | | 53 | 0 | 17 | 12 | 29 | 3 | | 50 | 0 | 1 | 0 | 1 |
| | | | | | | **36 (58.1 %)** | | | | | | | **2 (3.3%)** |

### Patient 3 Pancreatic Tumor

### Well-differentiated adenocarcinoma of the pancreas

### G2; T3; N1; M1

| **Control Morpholino** | | | | | | | **miR-10a Morpholino** | | | | | | |
|---|---|---|---|---|---|---|---|---|---|---|---|---|---|
| Day | Transplanted | Alive | | Migration | | **total >5** | Day | Transplanted | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **69** | 0 | 22 | 10 | 32 | 1 | | **66** | 0 | 2 | 0 | 2 |
| 2 | | 59 | 0 | 20 | 14 | 34 | 2 | | 53 | 0 | 4 | 0 | 4 |
| 3 | | 54 | 0 | 18 | 11 | 29 | 3 | | 49 | 0 | 3 | 0 | 3 |
| | | | | | | **34 (49.3%)** | | | | | | | **0 (6.1 %)** |

### RARα inhibitor treated Tumor Transplants

### Patient 1 Pancreatic Tumor

### poorly differentiated adenocarcinoma of the ampulla vateri with infiltration of the pancreas

### G3; pT3, pN1 mi (1/11) pM1

| **DMSO Control** | | | | | | | | **RAR**α **inhibitor** | | | | | |
|---|---|---|---|---|---|---|---|---|---|---|---|---|---|
| Day | Transplanted | Alive | | Migration | | **total >5** | Day | Transplanted | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **71** | 0 | 21 | 15 | 36 | 1 | | **65** | 1 | 2 | 0 | 2 |
| 2 | | 59 | 0 | 21 | 12 | 33 | 2 | | 62 | 0 | 2 | 0 | 2 |
| 3 | | 54 | 0 | 18 | 11 | 29 | 3 | | 58 | 0 | 1 | 0 | 2 |
| | | | | | | **36 (50.7%)** | | | | | | | **2 (3.1 %)** |

### Patient 2 Pancreatic Tumor

### Well-differentiated adenocarcinoma of the pancreas

### G2; T3; N1; M1

| **DMSO Control** | | | | | | | **RAR**α inhibitor | | | | | | |
|---|---|---|---|---|---|---|---|---|---|---|---|---|---|
| Day | Transplanted | Alive | | Migration | | **total >5** | Day | Transplanted | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **72** | 0 | 27 | 14 | 41 | 1 | | **70** | 1 | 3 | 0 | 3 |
| 2 | | 68 | 0 | 26 | 14 | 35 | 2 | | 67 | 1 | 2 | 0 | 2 |
| 3 | | 64 | 0 | 24 | 12 | 29 | 3 | | 63 | 0 | 1 | 0 | 1 |
| | | | | | | **41 (56.9%)** | | | | | | | **3 (4.3%)** |

### Patient 3 Pancreatic Tumor

### Well-differentiated adenocarcinoma of the pancreas

### G2; T3; N1; M1

| **DMSO Control** | | | | | | | **RAR**α **inhibitor** | | | | | | |
|---|---|---|---|---|---|---|---|---|---|---|---|---|---|
| Day | Transplanted | Alive | | Migration | | **total >5** | Day | Transplanted | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **68** | 0 | 22 | 12 | 34 | 1 | | **67** | 0 | 3 | 0 | 3 |
| 2 | | 59 | 0 | 21 | 12 | 33 | 2 | | 60 | 0 | 3 | 0 | 3 |
| 3 | | 54 | 0 | 19 | 11 | 30 | 3 | | 53 | 0 | 3 | 0 | 3 |
| | | | | | | **34 (50 %)** | | | | | | | **3 (4.5%)** |

Summary table for siRNA and morpholino experiments including controls

| **Experiment** | **PaTu-S (%migration)** | **PaTu-T (%migration)** | **PaTu-T +miR-10a Mo (%migration)** | **PaTu-T +control Mo (%migration)** | **PaTuS +miR-10a siRNA (%migration)** | **PaTu-S +control siRNA (%migration)** |
|---|---|---|---|---|---|---|
| 1 | 1.5 | 46.3 | 4.2 | 37.7 | 46.2 | 0 |
| 2 | 0 | 44.9 | 1.4 | 42.9 | 40 | 0 |
| 3 | 0 | 39.7 | 3 | 32.4 | 31.5 | 1.7 |
| 4 | 0 | 30 | - | - | - | - |
| **Average** | **0.375** | **38.18** | **2.86** | **37.67** | **39.23** | **0.57** |
| (SD) | (0.56) | (7) | (1.4) | (5.3) | (7.4) | (0.98) |
| **p-value** | | p=1.9 E-05 | | p=1.8 E-04 | | p=4.2 E-04 |

### Summary Table for RARα antagonist and HOXB1/B3 experiments

| **Experiment** | **+DMSO (%migration)** | **+RAR**α**inhibitor (%migration)** | **+RAR**α**inhibitor +HOXB1 (%migration)** | **+RAR**α**inhibitor +HOXB3 (%migration)** | **+RAR**α**inhibitor +HOXB1/3 (%migration)** | |
|---|---|---|---|---|---|---|
| 1 **PaTu-T** | 40.6 | 1.4 | 27.1 | 23.5 | 43.1 | |
| 2 **PaTu-T** | 42.9 | 2.9 | 23.4 | 27.3 | 48.5 | |
| **Average** | **41.8** | **2.2** | **25.3** | **25.4** | **45.8** | |
| (SD) | (1.2) | (0.8) | (1.9) | (1.9) | (2.7) | |
| **p-value** | | 3.9 E-04 | 1.7 E-05 | 4.4 E-04 | 1.3 E-05 | |
| | | | | | | |

| **Experiment** | **+control siRNA (%migration)** | **+HOXB1 siRNA (%migration)** | **+HOXB3 siRNA (%migration)** | **+ HOXB1/3siRNA (%migration)** | **+DMSO (%migration)** | **+RA** |
|---|---|---|---|---|---|---|
| 1 **PaTu-S** | 0 | 31.1 | 32.8 | 46.9 | 0 | 24.1 |
| 2 **PaTu-T** | 1.7 | 30.9 | 27 | 58.1 | 1.7 | 31.2 |
| **% Average** | **0.9** | **31** | **29.9** | **52.5** | **0.9** | **27.7** |
| (SD) | (0.9) | (0.1) | (2.9) | (5.6) | (0.9) | (3.6) |
| **p-value** | | 6.9 E-04 | 1.3 E-05 | 3 E-05 | | 1.0 E-05 |

| | | | | | | |
|---|---|---|---|---|---|---|
| A student t-test was performed and RARαinhibitor experiments in PaTu-T cells were compared to the DMSO control. In PaTu-S cells, all siRNA experiments were compared to control siRNA treated cells and RA treatment to DMSO treatment. | | | | | | |

**All tables for morpholino, siRNA, RA, RAR**α **antagonist and HOX experiments including controls (as summarized in the two tables above)**

### Injections of non-treated PaTu-S and PaTu-T cells

### Experiment 1

| **PaTu-S** | | | | | | | **PaTu-T** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **65** | 1 | 1 | 0 | 1 | 1 | | **69** | 6 | 25 | 6 | 31 |
| 2 | | 58 | 0 | 0 | 0 | 0 | 2 | | 63 | 5 | 23 | 6 | 29 |
| 3 | | 48 | 0 | 0 | 0 | 0 | 3 | | 55 | 5 | 22 | 6 | 28 |
| | | | | | | 1 (1.5 %) | | | | | | | **31 (46.3%)** |

| | | | | | | | | | | | | | |
|---|---|---|---|---|---|---|---|---|---|---|---|---|---|
| p=3.3279E-06 | | | | | | | | | | | | | |

### Experiment 2

| **PaTu-S** | | | | | | | **PaTu-T** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **63** | 2 | 0 | 0 | 0 | 1 | | **59** | 3 | 21 | 5 | 26 |
| 2 | | 55 | 0 | 0 | 0 | 0 | 2 | | 50 | 0 | 20 | 4 | 24 |
| 3 | | 49 | 0 | 0 | 0 | 0 | 3 | | 42 | 0 | 18 | 3 | 21 |
| | | | | | | **0 (0%)** | | | | | | | **26 (44.9%)** |

| | | | | | | | | | | | | | |
|---|---|---|---|---|---|---|---|---|---|---|---|---|---|
| p=4.15681 E-06 | | | | | | | | | | | | | |

### Experiment 3

| **PaTu-S** | | | | | | | **PaTu-T** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **55** | 2 | 0 | 0 | 0 | 1 | | **58** | 8 | 19 | 4 | 23 |
| 2 | | 43 | 0 | 0 | 0 | 0 | 2 | | 54 | 7 | 17 | 4 | 21 |
| 3 | | 39 | 0 | 0 | 0 | 0 | 3 | | 49 | 7 | 16 | 2 | 18 |
| | | | | | | **0 (0 %)** | | | | | | | **23 (39.7%)** |

| | | | | | | | | | | | | | |
|---|---|---|---|---|---|---|---|---|---|---|---|---|---|
| p=7.0939E-06 | | | | | | | | | | | | | |

### Experiment 4

| **PaTu-S** | | | | | | | **PaTu-T** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **61** | 0 | 0 | 0 | 0 | 1 | | **70** | 4 | 18 | 3 | 21 |
| 2 | | 57 | 0 | 0 | 0 | 0 | 2 | | 60 | 4 | 14 | 2 | 16 |
| 3 | | 54 | 0 | 0 | 0 | 0 | 3 | | 50 | 3 | 11 | 1 | 12 |
| | | | | | | **0 (0%)** | | | | | | | **21 (30%)** |

| | | | | | | | | | | | | | |
|---|---|---|---|---|---|---|---|---|---|---|---|---|---|
| p=0.0016474 | | | | | | | | | | | | | |

### Morpholinos:

### Experiment 1

| **PaTu-T + miR-10a morpholino** | | | | | | | **PaTu-T + control morpholino** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **71** | 1 | 3 | 0 | 3 | 1 | | **69** | 4 | 24 | 2 | 26 |
| 2 | | 68 | 0 | 2 | 0 | 2 | 2 | | 66 | 4 | 20 | 4 | 24 |
| 3 | | 66 | 0 | 1 | 0 | 1 | 3 | | 65 | 4 | 20 | 6 | 26 |
| | | | | | | **3 (4.2 %)** | | | | | | | **26 (37.7%)** |

| | | | | | | | | | | | | | |
|---|---|---|---|---|---|---|---|---|---|---|---|---|---|
| p=6.0679457E-06 | | | | | | | | | | | | | |

### Experiment 2

| **PaTu-T + miR-10a morpholino** | | | | | | | **PaTu-T + control morpholino** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **67** | 1 | 1 | 0 | 1 | 1 | | **70** | 7 | 26 | 2 | 30 |
| 2 | | 65 | 0 | 1 | 0 | 1 | 2 | | 66 | 6 | 23 | 2 | 25 |
| 3 | | 63 | 0 | 1 | 0 | 1 | 3 | | 63 | 6 | 20 | 2 | 22 |
| | | | | | | **1 (1.4 %)** | | | | | | | **30 (42.9%)** |

| | | | | | | | | | | | | | |
|---|---|---|---|---|---|---|---|---|---|---|---|---|---|
| p=0.000212398 | | | | | | | | | | | | | |

### Experiment 3

| **PaTu-T + miR-10a morpholino** | | | | | | | **PaTu-T + control morpholino** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **66** | 1 | 2 | 0 | 2 | 1 | | **71** | 6 | 20 | 3 | 23 |
| 2 | | 60 | 0 | 2 | 0 | 2 | 2 | | 64 | 6 | 20 | 3 | 23 |
| 3 | | 57 | 0 | 1 | 0 | 1 | 3 | | 65 | 5 | 19 | 3 | 22 |
| | | | | | | **2 (3 %)** | | | | | | | **23 (32.4%)** |

| | | | | | | | | | | | | | |
|---|---|---|---|---|---|---|---|---|---|---|---|---|---|
| p=7.59209E-07 | | | | | | | | | | | | | |

### miR-10a and control siRNAs:

### Experiment 1

| **PaTu-S + miR-10a siRNA** | | | | | | | **PaTu-S + control siRNA (miR-196a)** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **52** | 2 | 21 | 3 | 24 | 1 | | **54** | 0 | 0 | 0 | 0 |
| 2 | | 48 | 3 | 19 | 4 | 23 | 2 | | 50 | 0 | 0 | 0 | 0 |
| 3 | | 47 | 3 | 18 | 3 | 21 | 3 | | 48 | 0 | 0 | 0 | 0 |
| | | | | | | **24 (46.2 %)** | | | | | | | **0 (0%)** |

| | | | | | | | | | | | | | |
|---|---|---|---|---|---|---|---|---|---|---|---|---|---|
| p=6.8063E-06 | | | | | | | | | | | | | |

### Experiment 2

| **PaTu-S + miR-10a siRNA** | | | | | | | **PaTu-S + control siRNA (miR-196a)** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **60** | 4 | 18 | 3 | 21 | 1 | | **61** | 1 | 0 | 0 | 0 |
| 2 | | 54 | 4 | 19 | 5 | 24 | 2 | | 53 | 1 | 0 | 0 | 0 |
| 3 | | 47 | 4 | 18 | 3 | 21 **24 (40 %)** | 3 | | 49 | 0 | 0 | 0 | 0 **0 (0%)** |

| | | | | | | | | | | | | | |
|---|---|---|---|---|---|---|---|---|---|---|---|---|---|
| p=1.2632E-05 | | | | | | | | | | | | | |

### Experiment 3

| **PaTu-S + miR-10a siRNA** | | | | | | | **PaTu-S + control siRNA (miR-196a)** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **54** | 1 | 15 | 2 | 17 | 1 | | **58** | 1 | 1 | 0 | 1 |
| 2 | | 54 | 1 | 15 | 2 | 17 | 2 | | 56 | 1 | 1 | 0 | 1 |
| 3 | | 48 | 1 | 14 | 2 | 16 **17 (31.5 %)** | 3 | | 51 | 0 | 1 | 0 | 1 **1 (1.7%)** |

| | | | | | | | | | | | | | |
|---|---|---|---|---|---|---|---|---|---|---|---|---|---|
| p=6.12943E-06 | | | | | | | | | | | | | |

### Experiment 1 and 2

| **PaTu-T + DMSO** | | | | | | | **PaTu-T + DMSO** | | | | | | |
|---|---|---|---|---|---|---|---|---|---|---|---|---|---|
| Day | Injected | Alive | Migration | | | **total >5** | Day | Injected | Alive | Migration | | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **69** | 1 | 14 | 14 | 28 | 1 | | **70** | 2 | 16 | 14 | 30 |
| 2 | | 65 | 0 | 12 | 13 | 25 | 2 | | 66 | 1 | 16 | 13 | 29 |
| 3 | | 61 | 0 | 12 | 12 | 24 **1 (40.6%)** | 3 | | 59 | 1 | 14 | 11 | 25 **30 (42.9%)** |

### Experiment 1 and 2

| **PaTu-T + RAR**α**-inh** | | | | | | | **PaTu-T + RAR**α**-inh** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **70** | 0 | 1 | 0 | 1 | 1 | | **69** | 0 | 2 | 0 | 2 |
| 2 | | 67 | 0 | 0 | 0 | 0 | 2 | | 64 | 0 | 2 | 0 | 2 |
| 3 | | 63 | 0 | 0 | 0 | 0 | 3 | | 64 | 0 | 0 | 0 | 0 |
| | | | | | | **1 (1.4%)** | | | | | | | **2 ((2.9%)** |

### Experiment 1 and 2

| **PaTu-T +RAR**α**-inh +HOXB1** | | | | | | | **PaTu-T +RAR**α**-inh +HOXB1** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **66** | 1 | 12 | 4 | 16 | 1 | | **64** | 2 | 11 | 4 | 15 |
| 2 | | 61 | 2 | 11 | 3 | 14 | 2 | | 58 | 1 | 11 | 3 | 14 |
| 3 | | 58 | 0 | 10 | 3 | 13 **16(27.1 %)** | 3 | | 55 | 1 | 11 | 3 | 14 **15(23.4%)** |

### Experiment 1 and 2

| **PaTu-T +RAR**α**-inh +HOXB3** | | | | | | | **PaTu-T +RAR**α**-inh +HOXB3** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **68** | 1 | 11 | 5 | 16 | 1 | | **66** | 1 | 11 | 7 | 18 |
| 2 | | 61 | 0 | 10 | 4 | 14 | 2 | | 66 | 1 | 9 | 6 | 15 |
| 3 | | 56 | 0 | 9 | 4 | 13 | 3 | | 60 | 1 | 8 | 6 | 14 |
| | | | | | | **16(23.5 %)** | | | | | | | **18 (27.3%)** |

### Experiment 1 and 2

| **PaTu-T +RAR**α**-inh +HOXB1/B3** | | | | | | | **PaTu-T +RAR**α**-inh +HOXB1/B3** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **72** | 2 | 17 | 14 | 31 | 1 | | **66** | 3 | 18 | 14 | 32 |
| 2 | | 65 | 2 | 16 | 12 | 28 | 2 | | 65 | 2 | 17 | 14 | 31 |
| 3 | | 61 | 2 | 14 | 12 | 26 | 3 | | 65 | 2 | 16 | 12 | 28 |
| | | | | | | **31 (43.1 %)** | | | | | | | **32 (48.5%)** |

### Experiment 1 and 2

| **PaTu-S + HOXB1** | | | | | | | **PaTu-S + HOXB1** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **64** | 2 | 17 | 3 | 20 | 1 | | **68** | 1 | 16 | 5 | 21 |
| 2 | | 58 | 1 | 15 | 5 | 20 | 2 | | 58 | 1 | 13 | 4 | 17 |
| 3 | | 51 | 1 | 16 | 3 | 19 | 3 | | 51 | 0 | 12 | 5 | 17 |
| | | | | | | **20 (31.1 %)** | | | | | | | **21 (30.9%)** |

### Experiment 1 and 2

| **PaTu-S + HOXB3** | | | | | | | **PaTu-S + HOXB3** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **58** | 0 | 15 | 4 | 19 | 1 | | **63** | 1 | 12 | 5 | 17 |
| 2 | | 55 | 1 | 14 | 4 | 18 | 2 | | 53 | 2 | 10 | 4 | 14 |
| 3 | | 45 | 0 | 12 | 3 | 15 | 3 | | 49 | 2 | 3 | 4 | 7 |
| | | | | | | **19(32.8 %)** | | | | | | | **17 (27%)** |

### Experiment 1 and 2

| **PaTu-S + B1/B3** | | | | | | | **PaTu-S + B1/B3** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **64** | 2 | 18 | 12 | 30 | 1 | | **62** | 0 | 18 | 18 | 36 |
| 2 | | 54 | 2 | 16 | 10 | 26 | 2 | | 53 | 1 | 17 | 15 | 32 |
| 3 | | 47 | 2 | 16 | 9 | 25 | 3 | | 49 | 1 | 15 | 14 | 29 |
| | | | | | | **30 (46.9 %)** | | | | | | | **36 (58.1 %)** |

### Experiment 1 and 2

| **PaTu-S + DMSO** | | | | | | | **PaTu-S + DMSO** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **62** | 0 | 0 | 0 | 14 | 1 | | **59** | 1 | 1 | 0 | 1 |
| 2 | | 62 | 0 | 0 | 0 | 14 | 2 | | 57 | 1 | 1 | 0 | 1 |
| 3 | | 59 | 0 | 0 | 0 | 12 | 3 | | 56 | 1 | 1 | 0 | 1 |
| | | | | | | **0 (0 %)** | | | | | | | **1 (1.7%** |

### Experiment 1 and 2

| **PaTu-S + RA** | | | | | | | **PaTu-S + RA** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **58** | 1 | 11 | 3 | 14 | 1 | | **64** | 1 | 15 | 5 | 20 |
| 2 | | 58 | 0 | 10 | 4 | 14 | 2 | | 56 | 1 | 13 | 4 | 17 |
| 3 | | 49 | 0 | 19 | 3 | 12 | 3 | | 51 | 1 | 12 | 5 | 17 |
| | | | | | | **14 (24.1 %)** | | | | | | | **20 31.2%** |

### RARα and control siRNAs:

### Experiment 1 and 2

| **PaTu-T + control siRNA** | | | | | | | **PaTu-T + control siRNA** | | | | | | |
|---|---|---|---|---|---|---|---|---|---|---|---|---|---|
| Day | Injected | Alive | | Migration | | **total >5** | Day | Injected | Alive | Migration | | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **70** | 4 | 14 | 15 | 29 | 1 | | **73** | 2 | 17 | 15 | 32 |
| 2 | | 63 | 5 | 14 | 15 | 29 | 2 | | 71 | 3 | 16 | 14 | 30 |
| 3 | | 61 | 0 | 13 | 14 | 27 | 3 | | 64 | 3 | 13 | 10 | 23 |
| | | | | | | **29 (41.4%)** | | | | | | | **32 (43.8%)** |

### Experiment 1 and 2

| **PaTu-T + RAR**α **siRNA** | | | | | | | **PaTu-T + RAR**α **siRNA** | | | | | | |
|---|---|---|---|---|---|---|---|---|---|---|---|---|---|
| Day | Injected | Alive | | Migration | | **total >5** | Day | Injected | Alive | | Migration | | **total >5** |
| | | | <5 | 5 to 20 | >20 | | | | | <5 | 5 to 20 | >20 | |
| 0 | 80 | 80 | 0 | 0 | 0 | 0 | 0 | 80 | 80 | 0 | 0 | 0 | 0 |
| 1 | | **68** | 0 | 2 | 1 | 3 | 1 | | **65** | 0 | 2 | 2 | 4 |
| 2 | | 65 | 0 | 2 | 1 | 3 | 2 | | 62 | 0 | 2 | 2 | 3 |
| 3 | | 65 | 0 | 1 | 1 | 2 | 3 | | 61 | 0 | 2 | 2 | 0 |
| | | | | | | **3 (4.4%)** | | | | | | | **4 (6.1%)** |

### Human pancreatic cancer cell lines:

| | | **exp1** | **exp2** | **exp3** | **average** | **sd** |
|---|---|---|---|---|---|---|
| **Patu-T** | day1 | 40 | 52.4 | 45.6 | 46 | 4.3 |
| | day2 | 52.5 | 55 | 45 | 50.8 | 3.9 |
| | day3 | 58.8 | 57.9 | 55.6 | 57.4 | 1.6 |
| **Aspc1** | day1 | 16.7 | 19.4 | 12.3 | 16.1 | 2.6 |
| | day2 | 18.8 | 28 | 24.3 | 23.7 | 3 |
| | day3 | 19.4 | 28 | 28.7 | 16.8 | 4.7 |
| **Patu8902** | day1 | 16.3 | 17.8 | 21.2 | 22.8 | 2.5 |
| | day2 | 39.5 | 43.3 | 38.7 | 38.5 | 2 |
| | day3 | 39.4 | 50 | 38.7 | 42.7 | 4.9 |
| **Panc1** | day1 | 12.3 | 17.2 | 13.3 | 14.2 | 1.9 |
| | day2 | 19.1 | 17.2 | 20.7 | 19 | 1.2 |
| | day3 | 18.2 | 26.1 | 20.1 | 21.5 | 3.1 |
| **Mia Paca** | day1 | 22.2 | 23.5 | 25 | 23.6 | 0.9 |
| | day2 | 27.8 | 30 | 33.3 | 30.4 | 2 |
| | day3 | 41.2 | 30 | 33.3 | 34.8 | 4.2 |
| **Capan1** | day1 | 25.9 | 35.5 | 24 | 28.5 | 4.7 |
| | day2 | 27.6 | 28.8 | 29.5 | 28.6 | 0.7 |
| | day3 | 23.5 | 25 | 27.3 | 25.3 | 1.4 |
| **Capan2** | day1 | 18.2 | 17.8 | 19.5 | 18.5 | 0.7 |
| | day2 | 20.7 | 18.7 | 21.2 | 20.2 | 1.3 |
| | day3 | 32.1 | 25 | 28.9 | 28.7 | 2.5 |

| | | | | | | |
|---|---|---|---|---|---|---|
| **PaTu-S** cells as observed in the many experiments shown above are the only pancreatic cancer cells tested, which do not show metastatic behaviour. | | | | | | |

Experiment 1-3 (exp1-3): Invasion/migration of cells in % at the respective days. Day 1 refers to day 1 post injection (1 dpi). On average 80 zebrafish were injected per cell line per experiment.

### References

1. Jemal A, Siegel R, Ward E, Murray T, Xu J, Thun MJ. Cancer Statistics, 2007. CA Cancer J Clin 2007;57:43-66.
2. Johnson SM, Grosshans H, Shingara J, Byrom M, Jarvis R, Cheng A, Labourier E, Reinert KL, Brown D, Slack FJ. RAS Is Regulated by the let-7 MicroRNA Family. Cell 2005;120:635-647.
3. Esquela-Kerscher A, Slack FJ. Oncomirs [mdash] microRNAs with a role in cancer. Nat Rev Cancer 2006;6:259-269.
4. He L, Thomson JM, Hemann MT, Hernando-Monge E, Mu D, Goodson S, Powers S, Cordon-Cardo C, Lowe SW, Hannon GJ, Hammond SM. A microRNA polycistron as a potential human oncogene. Nature 2005;435:828-833.
5. Tavazoie SF, Alarcon C, Oskarsson T, Padua D, Wang Q, Bos PD, Gerald WL, Massague J. Endogenous human microRNAs that suppress breast cancer metastasis. Nature 2008;451:147-152.
6. Calin GA, Croce CM. MicroRNA signatures in human cancers. Nat Rev Cancer 2006;6:857-866.
7. Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature 2005;435:834-838.
8. Roldo C, Missiaglia E, Hagan JP, Falconi M, Capelli P, Bersani S, Calin GA, Volinia S, Liu C-G, Scarpa A, Croce CM. MicroRNA Expression Abnormalities in Pancreatic Endocrine and Acinar Tumors Are Associated With Distinctive Pathologic Features and Clinical Behavior. J Clin Oncol 2006;24:4677-4684.
9. Bloomston M, Frankel WL, Petrocca F, Volinia S, Alder H, Hagan JP, Liu C-G, Bhatt D, Taccioli C, Croce CM. MicroRNA Expression Patterns to Differentiate Pancreatic Adenocarcinoma From Normal Pancreas and Chronic Pancreatitis. JAMA 2007;297:1901-1908.
10. Ma L, Teruya-Feldstein J, Weinberg RA. Tumour invasion and metastasis initiated by microRNA-10b in breast cancer. Nature 2007;449:682-688.
11. Tanzer A AC, Kim CB, Stadler PF. Evolution of microRNAs located within Hox gene clusters. J Exp Zoolog B Mol Dev Evol. 2005;304:75-85.
12. Mainguy G, In der Rieden PMJ, Berezikov E, Woltering JM, Plasterk RHA, Durston AJ. A position-dependent organisation of retinoid response elements is conserved in the vertebrate Hox clusters. Trends in Genetics 2003;19:476-479.
13. Hong WK, Sporn MB. Recent Advances in Chemoprevention of Cancer. Science 1997;278:1073-1077.
14. Keidel S, LeMotte P, Apfel C. Different agonist- and antagonist-induced conformational changes in retinoic acid receptors analyzed by protease mapping. Mol. Cell. Biol. 1994;14:287-298.
15. Bertram JS, Vine AL. Cancer prevention by retinoids and carotenoids: Independent action on a common target. Biochimica et Biophysica Acta (BBA) - Molecular Basis of Disease 2005; 1740:170-178.
16. Kloosterman WP, Steiner FA, Berezikov E, de Bruijn E, van de Belt J, Verheul M, Cuppen E, Plasterk RHA. Cloning and expression of new microRNAs from zebrafish. Nucl. Acids Res. 2006;34:2558-2569.
17. Ott EB, te Velthuis AJW, Bagowski CP. Comparative analysis of splice form-specific expression of LIM Kinases during zebrafish development. Gene Expression Patterns 2007;7:620-629.
18. Marques I, Leito J, Spaink H, Testerink J, Jaspers R, Witte F, van den Berg S, Bagowski C. Transcriptome analysis of the response to chronic constant hypoxia in zebrafish hearts. Journal of Comparative Physiology B: Biochemical, Systemic, and Environmental Physiology 2008;178:77-92.
19. Woltering JM. MiR-10 Represses HoxB1a and HoxB3a in Zebrafish. PLoS ONE 2008;3:e1396.
20. Elsässer HP LU, Agricola B, Kern HF. Establishment and characterisation of two cell lines with different grade of differentiation derived from one primary human pancreatic adenocarcinoma. Virchows Arch B Cell Pathol Incl Mol Pathol. 1992;61:295-306.
21. Menke A, Philippi C, Vogelmann R, Seidel B, Lutz MP, Adler G, Wedlich D. Down-Regulation of E-Cadherin Gene Expression by Collagen Type I and Type III in Pancreatic Cancer Cell Lines. Cancer Res 2001;61:3508-3517.
22. Mayerle J, Schnekenburger J, Kruger B, Kellermann J, Ruthenburger M, Weiss FU, Nalli A, Domschke W, Lerch MM. Extracellular Cleavage of E-Cadherin by Leukocyte Elastase During Acute Experimental Pancreatitis in Rats. Gastroenterology 2005;129:1251-1267.
23. Marques, Weiss FU , Nitsche C, Partecke LI, Heidecke CD, Lerch MM , Bagowski C. Metastatic behaviour of primary human tumours in a zebrafish xenotransplantation model. BMC Cancer 2009;9:128..
24. Langenau DM, Traver D, Ferrando AA, Kutok JL, Aster JC, Kanki JP, Lin S, Prochownik E, Trede NS, Zon LI, Look AT. Myc-Induced T Cell Leukemia in Transgenic Zebrafish. Science 2003;299:887-890.
25. Stoletov K, Montel V, Lester RD, Gonias SL, Klemke R. High-resolution imaging of the dynamic tumor cell vascular interface in transparent zebrafish. Proceedings of the National Academy of Sciences 2007;104:17406-17411.
26. Park SW DJ, Rhee J, Hruban RH, Maitra A, Leach SD. Oncogenic KRAS induces progenitor cell expansion and malignant transformation in zebrafish exocrine pancreas. Gastroenterology 2008; 134:2080-90.
27. Nicoli S, Ribatti D, Cotelli F, Presta M. Mammalian Tumor Xenografts Induce Neovascularization in Zebrafish Embryos. Cancer Res 2007;67:2927-2931.
28. Topczewska JM, Postovit L-M, Margaryan NV, Sam A, Hess AR, Wheaton WW, Nickoloff BJ, Topczewski J, Hendrix MJC. Embryonic and tumorigenic pathways converge via Nodal signaling: role in melanoma aggressiveness. Nat Med 2006;12:925-932.
29. Nicoli S, Presta M. The zebrafish/tumor xenograft angiogenesis assay. Nat. Protocols 2007;2:2918-2923.
30. Lee, Seftor EA, Bonde G, Cornell RA, Hendrix MJC. The fate of human malignant melanoma cells transplanted into zebrafish embryos: Assessment of migration and cell division in the absence of tumor formation. Developmental Dynamics 2005;233:1560-1570.
31. Haldi M, Ton C, Seng W, McGrath P. Human melanoma cells transplanted into zebrafish proliferate, migrate, produce melanin, form masses and stimulate angiogenesis in zebrafish. Angiogenesis 2006;9:139-151.
32. Gould A, ltasaki N, Krumlauf R. Initiation of Rhombomeric Hoxb4 Expression Requires Induction by Somites and a Retinoid Pathway. 1998;21:39-51.
33. Jeanes A, Gottardi CJ, Yap AS. Cadherins and cancer: how does cadherin dysfunction promote tumor progression[quest]. Oncogene;27:6920-6929.
34. Nasevicius A, Ekker SC. Effective targeted gene /'knockdown/' in zebrafish. Nat Genet 2000;26:216-220.
35. Lam SH, Chua HL, Gong Z, Lam TJ, Sin YM. Development and maturation of the immune system in zebrafish, Danio rerio: a gene expression profiling, in situ hybridization and immunological study. Developmental & Comparative Immunology 2004;28:9-28.
36. Lowenfels AB, Maisonneuve P, Cavallini G, Ammann RW, Lankisch PG, Andersen JR, Dimagno EP, Andren-Sandberg A, Domellof L, The International Pancreatitis Study G. Pancreatitis and the Risk of Pancreatic Cancer. N Engl J Med 1993;328:1433-1437.

### Description of Figures and Tables

### Figure 1: MicroRNA-10a expression in pancreatic tumour cell lines and human specimen of pancreatic tumours or chronic pancreatitis

By Northern blot analysis we evaluated miR-10a expression in human pancreatic cancer cell lines **(A)** and in resection specimen **(B)** of normal human pancreas (NP), chronic pancreatitis (CP) and pancreatic tumours (PT). Densitometric analysis (Biorad GS800) showed average intensities of 4.2 (+/-0.5) for NP, 11.9 (+/-1.1) for PT and 12.7 (+/-0.6) for CP, suggesting a ∼ 3-fold elevated miR-10a expression in chronic pancreatitis and pancreatic tumour compared to normal pancreatic tissue. Control GAPDH expression, analysed by RT-PCR of the same RNA samples used in the northern blot, is shown in (C). Hematoxylyn-eosin (HE) stainings of representative histological sections of the utilized human tissue specimen are shown in (D) (NP: normal pancreas, CP: chronic pancreatitis, PT: pancreatic tumour).

### Figure 2: Uptake of miR-10a Morpholino into pancreatic cancer cells and tumour tissue

PaTu-T cells (**A-B**) and an ∼1 mm long tissue fragment of a human resection specimen of pancreatic tumour (**C-D**) were incubated for 24h in the presence of Endoporter with control Morpolino (**A**) or miR-10a-FITC Morpholino (**B-D**)**.** Uptake of FITC-labeled Morphlino was analysed by confocal (**A-B** middle panels) and fluorescence microscopy (**C-D** middle panels). Analysis of bright field (**A-D** left panels) and overlay images (**A-D** right panels) demonstrates uptake efficiencies of >98% in PaTu-T cells. Notably significant uptake of Morpholino into cells within the resection specimen was also detected even though the transfection efficiency could not be determined. No significant autofluorescence of tumour tissue was observed (Supplemental Fig. 5).

### Figure 3: Inhibition of miR-10a function by miR-10a specific Morpholinos in pancreatic cancer cells

PaTu-T cells were transfected with a sensor construct containing a green fluorescence protein (GFP) coding sequence followed by multiple miR-10a target sites. As demonstrated in the schematic representation, GFP is expressed from this sensor construct only in the absence of miR-10a (**A**)**.** Due to expression of miR-10a in PaTu-T cells, miR-10a can bind to the 3' target sites and suppress GFP expression (**B**). This suppressive effect of miR-10a can be released by addition of a specific miR-10a Morpholino which itself targets miR-10a and prevents its binding to the sensor construct (C). Following transfection, PaTu-T cells were incubated for 24 h with control or miR-10a Morpholinos and investigated by fluorescence microscopy (upper panel). In contrast to the control cells, flourescencent cells were only detected in miR-10a Morpholino treated PaTu-T cells. A low transfection efficiency of PaTu-T cells is responsible for only limited numbers of fluorescent cells (tested with a GFP-expression construct; data not shown).

### Figure 4: MicroRNA-10a is required and sufficient for tissue invasion and metastasis formation of pancreatic tumour cells

PaTu-T cells were incubated with control and miR-10a Morpholinos, stained with fluorescent CM-Dil and transplanted into the yolk of zebrafish embryos. Blockage of miR-10a by specifc Morpholino was found to inhibit migration of PaTu-T cells in xenotransplanted zebrafish (**A**) whereas PaTu-T cells treated with a control Morpholino still invaded the embryo and formed metastases (**B**). To determine whether miR-10a is able to induce tissue invasion and migration of non-invasive PaTu-S cells, we incubated the cells with synthetic miR-10a siRNA and, as a control, synthetic miR-196a siRNA (control-siRNA). We observed tissue invasion and migration in miR-10a siRNA (**C**), but not in control-treated cells (**D**), which remained in the yolk. Images (**A-D**) were taken by high resolution fluorescence stereomicroscopy at 3 days post fertilization and 1 day post injection (dpi). The presence of representative tumour cells and micrometastases at 3 dpi is shown in DIC microscopy pictures overlayed with a fluorescence image (**E**) and HE staining (**F**) of a transversal section of zebrafish liver. Arrows (in **E-F**) point to micrometastases in the liver (see also Supplemental Fig. 4).

### Figure 5: RAR antagonists inhibit miR-10a expression and migration of pancreatic cancer cells

PaTu-T cells were incubated as indicated, with DMSO (Co), 200 nM Ro-41-5253 (RARα inh), control-Morpholino, miR-10a Morpholino, miR-196 siRNA (Co), or RARα siRNA and PaTu-S cells were co-incubated with 1 µM retinoic acid (RA) and control siRNA, RARα siRNA or Ro-415253 and miR-10a expression was evaluated by Northern blot analysis (**A**). Densitometric analysis (Biorad GS800) revealed in PaTu-T cells a 9.4 fold reduction of miR-10a expression following Ro-415253 treatment, a 10.1 fold reduction by RARα siRNA treatment and a 1.3 fold reduction following miR-10a Morpholino treatment. In contrast miR-10a expression was upregulated by 7.2 fold in PaTu-S cells following treatment with retinioic acid (1µM final concentration). This upregulation was completely blocked by RARα siRNA or RARα inhibitor. RT-PCR control of GAPDH was used as a loading control (not shown). Dose response curves for RA and the RARα antagonist and the RT-PCR control for RARα siRNA treatment are shown in Supplemental Fig. 1. The effect of Ro-415253 on cell migration was analysed in an *in vitro* migration assay in PaTu-T cells. Representative images demonstrate significant inhibition of cell migration following Ro-415253 treated in comparison to control (DMSO) cells (**B**). The regulatory function of miR-10a on expression of proteins of the cadherin/catenin complex was demonstrated in PaTu-S and ASPC-1 cells. Western blot analysis of total lysates of PaTu-S cells and ASPC-1 cells showed reduced expression of E-Cadherin, β-Catenin and α-Catenin (only PaTu-S) following treatment with indicated concentrations of miR-10a siRNA (**C**). An actin re-blot confirmed equal loading.

### Figure 6: Suppression of HOXB1 and HOXB3 confers invasiveness and metastatic behaviour

PaTu-S and PaTu-T cells were treated (24h) as indicated with specific siRNAs against HOXB1 (B1) and HOXB3 (B3) (Co: DMSO control) in the presence or absence of Ro-41-5253. Migration of these cells was analysed after transplantation into zebrafish embryos (see Table 1) and downregulation of HOXB1 and HOXB3 expression in these cells was confirmed by PCR analysis (A).

RT-PCR-analysis revealed high expression levels of HOX genes B1 and B3 in PaTu-S cells and low expression in PaTu-T cells. Inverse regulation of HOX expression in response to 24h incubation with 200 nM Ro-41-5253 (RARα inh) or 1 µM retinoic acid (RA) is demonstrated (left side, DMSO: solvent control). A similar regulation is seen following incubation with miR-10a Morpholinos or with miR-10a siRNAs (right side; Control: control Morpholino or control-siRNA) (B). Results are normalized to β-actin expression and similar results were obtained in three independent experiments. The schematic model in (C) summarizes our findings: RA stimulates dimerization of RAR/RXR retinoid receptors which bind to retinoic acid response elements (RAREs) in the regulatory region of miR-10a and trigger increased miR-10a expression. Suppression of HOXB1 and HOXB3 by miR-10a then promotes invasion and metastatic behaviour (upper panel). RA stimulation, miR-10a overexpression or suppression of HOXB1 and HOXB3 (by siRNAs) are equally sufficient to confer metastatic behaviour to these cells. Transcriptional repression of miR-10a by a selective RARα inhibitor (Ro-415253) releases the suppression of HOXB1 and HOXB3 and prevents invasiveness and metastatic behaviour (lower panel). The inhibitory function of Ro-415253 can be overruled by a suppression of HOXB1 and HOXB3 with siRNAs.

### Table 1: Retinoic acid, miR-10a and HOX Genes B1 and B3 control metastatic behaviour of pancreatic cancer cells

PaTu-T cells were incubated with miR-10a Morpholino, Ro-41-5253 (RARα inh) or combinations of RARα inh with specific siRNAs against HOXB1 and HOXB3. PaTu-S cell on the other hand were incubated with miR-10a siRNA, RA or the specific siRNAs against HOXB1 and HOXB3 alone. Migration of these cells was analysed after transplantation into zebrafish embryos. The averages of observed metastasised embryos (in %) are shown including standard deviations (SD). All experiments were performed 2-4 times and experimental details including p-values are given in Supplemental Table 1. Downregulation of HOXB1 and HOXB3 expression by siRNAs in these cells was confirmed by PCR analysis (see Fig. 6A).

### Table 2: Metastatic behaviour of human tumour explants treated with morpholino and RARα inhibitor

Small tissue fragments from two well differentiated ductal adenocarcinoma resection specimen of the Pancreas head (2 and 3) were incubated with miR-10a Morpholino or RARα inhibitor and, following staining with carbocyanine dye (CM-Dil), transplanted into zebrafish embryos (Controls: DMSO and control-Morpholino). Formation of micrometastases in the fish was assessed at 3 dpi by analysis of present cell aggregates (including daughter cells) outside the yolk sac. In both miR-10a Morpholino or RARα-antagonist treated specimen a significant decrease in metastatic behavior was detected (p< 0.001, student t-test). Tumour grading and classification was done according to histopathological examination. A representable HE-stained tumour section is shown in Fig.1 D.

### Supplemental Figure 1:

Supplemental Figure 1: (A) Dose dependent repression of miR-10a expression in PaTu-T cells by RARα inhibitor Ro-415253. PaTu-T cells were incubated for 24h with indicated concentrations of Ro-415253 and miR-10a expression was evaluated by northern blot analysis (upper panel). GAPDH loading control is shown in the lower panel. (8) Dose dependent induction of miR-10a expression in PaTu-S cells by retinoic acid (ATRA). Patu-S cells were incubated tor 24h with indicated concentrations of ATRA and miR-10a expression was evaluated by northern blot analysis (upper panel). GAPDH loading control is shown in the lower panel. (C) Specific "knockdown" of RARα in PaTu-Tcells by RARα siRNA. RT-PCR from total RNA of RARα or control siRNA (Co) treated PaTu-T cells (24h) confirms specific knockdown of RARa. RT-PCR of GAPDH control is shown in the lower panel.

### Supplemental Figure 2: Binding of RARα to a retinoc response element (RARE) in the vicinity of miR-10a.

(A) Chromatin immunoprecipitation (CHIP)analysis demonstrates direct binding of retinoic acid receptor RARa to a DR5 type 10 retinoic acid response element (RARE) near the locus of the miR-10a gene on chromosome 17. Binding of RARa was only detectable in PaTu-T cells, but not in non-invasive PaTu-S cells. In negative controls RAR-antibodies were omitted (noAb), in positive controls (input) a fraction of the genomic DNA was directly submitted to PCR analysis. (see method). (B) Schematic representation of the HoXB cluster on Chromosome 17. The localization of miR-10a, HoXB4 Exons 1 and 2 and the DR5 RARE are shown and black arrows indicate the localization of utilized primers. The DR5 RARE has previously been demonstrated to act as a neural enhancer for HoxB4 expression in mouse neural tube (Reference 35) and thus is a likely regulator for miR-10a.

Method: Chromatin immunoprecipitation (ChIP) assays were performed using reagents from Upstate Biotechnology according to the manufacturer's instructions with some modifications. Cells were cross-linked for 10 minutes at 37 C (DMEM, 1% formaldehyde) and subsequently lysed (50 mM Tris-HCl, pH 8.1, 10 mM EDTA, 1% SDS, incl. protease inhibitors) at a final concentration of 106 cells per 200 µl. Lysates were sonicated on ice by 7x10s bursts at 30% power using a Bandelin Sonicator UW 2200 (Bandelin electronic, Berlin, Germany) and centrifuged for 10 minutes at 14.000 rpm at 4C to remove cell debris. An aliquot of 200 µl was taken for input control. After pre-clearing ON with protein A agarose 300µl lysate were diluted in dilution buffer (incl protease inhibitors) to a final volume of 2 ml and 1 ml aliquots were incubated with 4 µl of antibody RARα (sc-551) (Santa Cruz Biotechnology, Inc.) and Protein A agarose overnight at 4 C. Agarose was pelleted by brief centrifugation and washed subsequently with low salt immune complex wash buffer [20 mM Tris-HCl, pH 8.1, 150 mM NaCl, 2 mM EDTA, 1% Triton X-100, 0.1% SDS], high salt immune complex wash buffer [20 mM Tris-HCl, pH 8.1, 500 mM NaCl, 2 mM EDTA, 1% Triton X-100, 0.1% SDS], LiCl immune complex wash buffer [10 mM Tris-HCl, pH 8.1, 1 mM EDTA, 0.25 M LiCl, 1% Nonidet P-40, 1 % deoxycholic acid] and twice with TE buffer [10 mM Tris-HCl, 1 mM EDTA]. The pellet was finally dissolved in 500 µl elution buffer [0.1 M sodium-hydrogen-carbonate, 1% SDS] to elute protein-DNA complexes and de-crosslinked in 0.2 M NaCl by heating at 65 C for 4 hours. After proteinase digestion (10 mM EDTA, 40 mM Tris-HCl, pH 6.5, ca. 60 µg Qiagen proteinase K) DNA was recovered in 60 µl water using the QIAEX-II Gel Extraction Kit (Qiagen, Hilden, Germany). Both, immunoprecipitated and input DNA was subjected to PCR (95 C for 5 minutes, 66 C for 45 seconds and 72 C for 45 seconds for 40 cycles). Primer sequences for DR5 type 10 were as follows: 5'-ACA GAC AAG GTG GAC TGA TGC AG-3' (sense) and 5'-CAT TCA GGT CGG CTG CAG AGC CC-3' (antisense) to amplify a 192 bp product.

### Supplemental Figure 3:

Supplemental Figure 3: Cell cycle analysis of RARα siRNA or Ro-415253 treated PaTu-T cells. Cells were treated with either DMSO control (A), RARα siRNA (B) or 200 nM Ro-415253 (C) and fixed after 24h with 70% EtOH. Following propidium iodine (PI) staining the cells were analysed by a LSR2 FACS sorter (Becton Dickinson). The graphs are representative examples of three experiments with comparable outcome. Gate P2 indicates cells in G0/1 phase, gate 3 represents cells in S phase and gate 4 represents Cells in M/G2 phase. No differences in cell cycle distribution after treatment with RARα siRNA or Ro-415253 were observed.

### Supplemental Figure 4:

Supplemental Figure 4: Formation of micrometastases by PaTu-T cells in zebrafish embryos. CM-Dil-stained Patu-T cells and micrometastases can be detected 4 days post transplantation in cross sections of zebra fish embryos in differential interference contrast (Die) images (A,B,G,K), HE stains (D,E,F,H,L,M) and fluorescence images (C,J). Formation of micrometastases can be seen in the exocrine pancreas (A box1 and higher magnifications F,G), close to the gut (A box2 and C ,E) and in the pronephric duct (H box3 and I-M). B and D represent higher magnification pictures of A.

### Supplemental Figure 5:

Supplemental Figure 5: Confirmation of cellular uptake of Morpholino by resected pancreatic tumour specimen. Small fragments of human tumour specimen (A, bright field) were incubated for 24h with FITC-labeld miR-10a Morpholino. After washing with PBS uptake of Mo-FITC was investigated in a fluorescence microscope (Dual interference contrast microscope, Axiocam, Carl Zeiss, Jena). FITC signals in (B) verify uptake of fluorescent Morpholino by the tissue specimen. Fluorescence images of the tumour specimen previous to Mo-FITC incubation (C) confirm that the FITC signal seen in (B) is not due to tissue autofluorescence. D, E and F are higher magnifications of corresponding images A, B and C.

### Supplemental Table 1:

Table showing detailed results for all transplantation experiments (including Tumor Transplants, PaTu-S and PaTu-T cells as well as additional human pancreatic cancer cell lines treated with siRNAs, Morpholinos, RA and RARα antagonist).

## Claims

1. RAR antagonists or miR-10a inhibitors or inhibitors of HOXB1 and/or HOXB3 repressors for the treatment of pancreatic cancer.

2. RAR antagonists or miR-10a inhibitors or inhibitors of HOXB1 and/or HOXB3 repressors according to claim 1 for the prevention or reduction of metastasis in pacreatic cancer.

3. RAR antagonists according to claim 1 or 2, **characterized in that** the RAR antagonist is selected from Ro-41-5253 and its derivatives.

4. RAR antagonists according to any one of the claims 1-3, **characterized in that** the RAR antagonist is selected from the group of retinoid-like tricyclic compounds which bind with high affinity to RARs and are potent antagonists of retinoic acid function.

5. miR-10a inhibitors according to claim 1 or 2, **characterized in that** the miR-10a inhibitor is the miR-10a antisense sequence CACAAATTCGGATCTACAGGGTA.

6. miR-10a inhibitors according to any one of the claims 1, 2 or 5, **characterized in that** the miR-10a inhibitor is the miR-10a antisense sequence, wherein one or more of these 23 nucleic acids are deleted or substituted or wherein one or more nucleic acids are added and which still inhibit the miR-10a sequence.

7. Inhibitors of HOXB1 and/or HOXB3 repressors according to claim 1 or 2, **characterized in that** they are selected from the group of antisense oligonucleotides, which compriseshort DNA or RNA sequences designed to be complementary to the target sequence such as siRNA, Morpholins, vivo-Morpholins, and specifically interfere with the repression of HOX genes B1 and B3.

## Amended claims

### Amended claims in accordance with Rule 137(2) EPC.

**1.** MiR-10a inhibitors or inhibitors of HOXB1 and/or HOXB3 repressors for the treatment of pancreatic cancer.

**2.** MiR-10a inhibitors or inhibitors of HOXB1 and/or HOXB3 repressors for use according to claim 1 for the prevention or reduction of metastasis in pancreatic cancer.

**3.** MiR-10a inhibitors for use according to claim 1 or 2, **characterized in that** the miR-10a inhibitor is the miR-10a antisense sequence CACAAATTCGGATCTACAGGGTA.

**4.** Inhibitors of HOXB1 and/or HOXB3 repressors for use according to claim 1 or 2, **characterized in that** they are selected from the group of antisense oligonucleotides, which comprise DNA or RNA sequences designed to be complementary to a target sequence such as siRNA, Morpholins, vivo-Morpholins, and specifically interfere with the repression of HOX genes B1 and B3.
